# EUROPEAN PATENT APPLICATION

(11) **EP 4 775 145 A1**
(43) Date of publication of application: **15.07.2026**
(21) Application number: 25151344.6
(22) Date of filing: 10.01.2025
(51) Int. Cl.: A61B 5/251

(54) **CONDUCTIVE UNIT WITH SOLID ELECTROLYTE**

(71) Applicant: Nahtlos AG, 9014 St. Gallen (CH)
(72) Inventor: SCHMID, Michel, 9011 St. Gallen (CH); NÄF, José, 9443 Widnau (CH)
(74) Representative: Kaminski Harmann Patentanwälte AG (Schweiz)

(57) **Abstract**

The invention relates to a conductive unit 30 for a contact system 1, in particular electrode, wherein the conductive unit 1 is provided to be contacted with a skin of a person such that an electrical contact to the skin of the person by the contact system 1 is provideable, wherein the conductive unit 30 comprises an electrolyte 100 having a solid aggregate state.

## Description

### FIELD OF THE INVENTION

The present invention relates to a conductive unit for a contact system, a method for producing the conductive unit, the respective contact system and a method of activating the contact system.

### BACKGROUND OF THE INVENTION

The measurement of electrical body signals such as ECG (electrocardiogram), EEG (electroencephalogram), EMG (electromyogram) are widely used measuring methods for deriving body signals. Today the ECG is used in the medical field typically with Ag/AgCI gel electrodes, which are glued to the skin around the chest area. These electrodes are more or less a standard and are used worldwide for ECG measurements in the medical field.

A serious problem with dry electrodes is the movement artifacts due its high skin-electrode impedance, which is further influenced by variability in skin properties, such as moisture or sebum production. With dry skin, movement is even more critical, i.e. the slightest movement of the arms or chest can lead to extremely high artifacts. To filter out a reasonable ECG signal from these is extremely difficult.

US 6,263,226 B1 describes a disposable electrode design that relies on a conductive gel placed between the skin and the electrode's conductive element. Although this approach can ensure a stable initial contact, the gel may dry out or degrade over time, potentially reducing the reliability and consistency of the electrical interface during extended use.

From EP 510 786 A1 an electrode is known, which is based on a hydrogel layer, which is brought together in combination with a non-conductive material layer and is connected to a reusable clamp. No moistening device is provided as the hydrogel layer provides the moistening function. The device of EP 510 786 A1 is based on the principle of an electrolyte layer based on hydrogel.

To provide a stable electrical contact between the electrode and the skin, an electrolyte-containing (hydro)gel is thus applied directly to the skin in some prior art electrodes, such as the electrodes described above, before the electrode is applied. However, this approach has the serious disadvantage that the additional step of applying the electrolyte gel is necessary for the electrode to function properly. It is therefore not possible to wear the electrode on the skin and activate the electrode directly on the skin at the start of the measurement.

Other prior art electrodes solve the problem of moistening the skin with an electrolyte solution / electrolyte gel to ensure proper transmission of the electrical signal by having a sponge-like tissue in the electrode which is saturated with an electrolyte solution / electrolyte gel. To activate the electrode, the electrolyte solution / electrolyte gel is then squeezed out of this sponge. This is a disadvantage of these prior art electrodes, as the sponge must be prevented from drying out in order for the electrode to function as desired.

So that the sponge does not dry out before the electrode is used, this sponge is often surrounded by at least one layer that protects against evaporation in prior art electrodes. However, this has the disadvantages that the protective layers have to be laboriously removed before the electrode is applied or that the electrolyte solution has to penetrate these protective layers until it reaches the patient's skin from the sponge, whereby the electrolyte remains in the sponge and in these layers and dries out there. Accordingly, the electrolyte concentration of the electrolyte solution / electrolyte gel directly on the skin is significantly reduced compared to the initial concentration in the sponge. This in turn has a negative effect on the transmission of the electrical signal. A further disadvantage is that the electrolyte solution can be pushed sideways away from the contact surface of the electrode on the skin under the pressure on the sponge.

The approaches of above have in common that they represent comparatively complex electrodes and/or have difficulties in providing a stable, interference-free electrical contact with the skin, especially for long-term applications where a gel can dry out.

### OBJECT OF THE INVENTION

Accordingly, it is an object of the invention to propose an improved conductive unit for a contact system, a method for producing the conductive unit, the respective contact system and a method of activating the contact system in which the described disadvantages of the prior art can at least be reduced.

It is an object of the invention to provide a conductive unit for a contact system and the respective contact system for providing an improved electric contact to the skin of a person.

It is a further object of the invention to provide an improved contact system which is easy to use.

These objects are achieved by realizing at least part of the features of the independent claims. Features which further develop the invention in an alternative or advantageous manner are described in the dependent patent claims.

### SUMMARY OF THE INVENTION

The present invention relates to a conductive unit for a contact system, in particular electrode, wherein the conductive unit is provided to be contacted with a skin of a person such that an electrical contact to the skin of the person by the contact system is provideable. The conductive unit according to the invention comprises an electrolyte having a solid aggregate state.

The solid aggregate state of the electrolyte can be determined under a microscope, for example, whereby the electrolyte can be seen as crystals in the conductive unit. Furthermore, the solid aggregate state of the electrolyte can be determined using X-ray diffraction (XRD), in particular X-ray powder diffraction (XRPD). XRD can also be used to determine other physical properties such as crystallite size (diameter), crystallite orientation, residual stress, etc.

In one exemplary embodiment of the inventive conductive unit, the conductive unit can comprise a conductive layer, wherein the conductive layer can comprise an electrically conductive element, in particular electrically conductive threads or electrically conductive synthetic material, in particular made of metal or metallized synthetic material, in particular yarns, and/or is provided to be contacted with the skin of the person. Optionally, the conductive layer can comprise the electrolyte having a solid aggregate state.

In a further exemplary embodiment of the inventive conductive unit, the conductive unit can further comprise an electrolyte storage layer, wherein the electrolyte storage layer can comprise the electrolyte having a solid aggregate state.

In a further exemplary embodiment of the inventive conductive unit, the electrolyte can be dissolvable in a fluid by wetting or humidifying the conductive unit with the fluid to provide a solution of the electrolyte so that the solution of the electrolyte is providable to the skin of the person from the conductive unit. By means of such a provision of the solution of the electrolyte from the conductive unit onto the skin of the person, a reduction of a skin resistance and thus an improved electrical contact to the skin of the person by the contact system is providable.

Optionally, the electrolyte can be already comprised by the fluid prior to the wetting or humidifying of the conductive unit with the fluid.

In a further embodiment, the conductive layer can comprise or can be made from a textile or a polymeric layer.

In a further embodiment, the electrolyte storage layer can comprise or can be made from a textile, cotton, cellulose, paper, in particular blotting paper, or a polymeric layer.

In a further embodiment, the conductive unit can comprise a fluid and can be provided in a moistened, humidified, or wetted state.

In a further embodiment, the electrolyte can comprise at least one alkaline metal salt, in particular sodium chloride or potassium chloride, and/or at least one alkaline earth metal salt, in particular magnesium chloride or calcium chloride.

In the case of e.g. sodium chloride as electrolyte, the solid aggregate state of the electrolyte is characterized, for example, by the fact that the sodium chloride is present as small (salt) crystals in the conductive unit. These (salt) crystals can be viewed under a microscope.

In a further embodiment, a hydrophobicity of the conductive layer can be reduced by the electrolyte compared to a hydrophobicity of a conductive layer comprising the same material as the conductive layer but without the electrolyte (in the inventive production process for the conductive unit described below, this conductive layer and/or the electrolyte storage layer without electrolyte is also referred to as the conductive unit precursor).

The hydrophobicity of the conductive layer is a measure of a size of a contact angle between the conductive layer and the fluid which can be determined by means of static contact angle measurement.

Since the contact systems, in particular electrodes, for which conductive layers are used are intended for providing an electrical contact to the skin of a person, it was necessary for prior art conductive layers to be made of a hydrophilic material in order to ensure sufficient absorption of a fluid, e.g. water, and good distribution of the fluid on the skin. The use of hydrophobic materials for conductive layers would have resulted in the fluid, e.g. water, not penetrating the conductive layer or even beading off the conductive layer, which would have prevented the fluid from reaching the skin and thus interfered with the transmission of an electrical signal.

However, this embodiment of the inventive conductive unit has the advantage that many more materials, in particular also (rather) hydrophobic materials, can be used for the production of the conductive layer, since the hydrophobicity of the conductive layer is reduced by the solid electrolyte present therein, in other words the conductive layer becomes more hydrophilic.

The present invention further relates to a method for producing a conductive unit according to any one of the previous described embodiments, the method comprising the step of applying the electrolyte to a conductive unit precursor, wherein the electrolyte is applied to the conductive unit precursor by mechanical application of the electrolyte being in the solid aggregate state, and/or by means of a production fluid, in particular wherein the electrolyte can be dissolved in the production fluid. Optionally, the conductive unit precursor can correspond to the conductive layer without the electrolyte and/or the electrolyte storage layer without the electrolyte (100).

For example, the electrolyte can be applied on / to the conductive unit precursor in a solid aggregate state and then mechanically worked into / rubbed in / massaged in the conductive unit precursor. However, the electrolyte can also be sprayed onto the conductive unit precursor using a stream of (compressed) air, for example. The electrolyte would then be in a solid aggregate state again, but would be applied to the conductive unit precursor by means of the production fluid air. This method of applying the electrolyte can be compared to spraying sand from a sandblaster. A further exemplary embodiment of the method according to the invention provides that the electrolyte is first dissolved in a liquid production fluid, e.g. water, and this electrolyte solution is then brought into contact with the conductive unit precursor. For example, the conductive unit precursor can be immersed in a basin filled with said electrolyte solution. If the electrolyte is applied to the conductive unit precursor by means of such an electrolyte solution, the production process according to the invention requires a drying step as described below, in which the production fluid is removed again, leaving the electrolyte in the conductive unit precursor and thus forming the conductive unit.

In one exemplary embodiment of the inventive method, the production fluid can comprise at least one of the following: a gas, in particular nitrogen or air, a liquid, in particular water, a gel, in particular a hydrogel, a solution, in particular a water-based solution or an alcohol-based solution, an emulsion, or oil.

In a further exemplary embodiment of the inventive method, if the electrolyte is applied to the conductive unit precursor dissolved in the production fluid, the method can further comprise the step of evaporating the production fluid, in particular wherein the evaporation of the production fluid is provided by exposing the conductive unit precursor to ambient air, and/or exposing the conductive unit precursor to a gas flow, and/or applying a negative pressure to the conductive unit precursor, and/or increasing a temperature around the conductive unit precursor.

The present invention further relates to a contact system, in particular electrode, for providing an electric contact to the skin of a person, the contact system comprising a conductive unit according to any one of the previous described embodiments, and a reservoir, the reservoir comprises an encapsulation. This encapsulation (at least in an undamaged state) comprises a fluid, and provides separation of the fluid from the conductive unit, and is configured to be broken upon defined external influence. The conductive unit is arranged relative to the encapsulation so that - in the event of a break of the encapsulation - wetting of the conductive unit by the fluid is provided.

In other words, in the contact system of the present invention, water is encapsulated and is only released when required by pressing a button. The electrolyte is integrated into the contact system (electrode) in a dry state and is only converted into a liquid electrolyte when activated, which effectively reduces the skin contact resistance. This technology makes both the manufacture and storage of the contact system (electrode) considerably easier.

The contact system of the present invention has at least the advantage over the gel electrodes of the prior art described above that it is not necessary to specially protect a gel from drying out, which in turn would result in the electrode no longer being able to conduct the signals stably. Dry electrodes generally have a poorer signal quality. In the case of variants where the adhesive is electrically conductive, there is also the problem that these are quickly detached from the body when sweating.

Under the term to be broken upon defined external influence (or exposure) is understood not only the physical breaking, i.e. the bursting of the surface of the encapsulation under the influence of a force (for example triggered by bending or pressing in the surface with the fingers or hitting the surface with a kind of hammer), but any way known to the skilled person to open a surface by an external influence or exposure.

The encapsulation can also be ripped open, for example, by a pulling movement (for example, with two fingers), whereby the surface of the encapsulation can be pulled directly or on a part protruding from the surface of the encapsulation, i.e. a kind of flap.

Breaking of the encapsulation can also be achieved by scratching or cutting or piercing the encapsulation by means of an edge or blade or tip (i.e. the breaking element) or any other protruding part attached, for example, to a tilting element or a sliding element.

In one exemplary embodiment of the inventive contact system, the contact system can further comprise a fluid absorption unit, in particular a sponge, wherein the fluid absorption unit is arranged between the conductive unit and the encapsulation, so that - in the event of a break of the encapsulation - wetting of the fluid absorption unit by the fluid is provided, so that the fluid absorption unit absorbs the fluid and then successively releases the fluid on / to the conductive unit.

In a further exemplary embodiment of the inventive contact system, the reservoir can comprise a transmissive layer, wherein the transmissive layer can be configured to provide defined transmission of vaporous parts of the fluid with a defined transmission rate. Further, the encapsulation, the transmissive layer and the conductive unit can be arranged relative to each other so that - in the event of a break of the encapsulation - wetting of the transmissive layer by the fluid is provided and humidifying of the conductive unit by transmission of the vaporous parts of the fluid through the transmissive layer is provided.

In particular, the transmissive layer is embodied to provide the defined transmission rate. The transmission rate can be adjusted to a particular fluid which is comprised by the reservoir, i.e. the transmissive layer and the fluid can be chosen or designed relative to each other so that a particular amount of the vaporous parts of the fluid can pass the transmissive layer.

Due to the arrangement and designs of the transmissive layer and the conductive unit, the conductive unit can be kept humid over a comparatively long time period. This is provided by defined diffusion of vapourous parts of the fluid, i.e. out of the reservoir by passing the transmissive layer and wetting the conductive unit. In other words, there can be provided continuous transmission of vaporous parts of the fluid.

In particular, the configuration of the transmissive layer is adjusted with respect to the type of fluid so that the wetting of the conductive unit is provided as desired. In particular, the transmissive layer comprises particularly designed perforations or respectively tight weaving or knitting.

The term "vaporous parts of the fluid" is used to illustrate that the transmissive layer is a membrane or similar that allows substances in the gaseous aggregate state, e.g. gases such as oxygen, nitrogen, argon, air, etc., or, for example, water vapor, to pass through / transmit / permeate, but does not allow substances in the liquid aggregate state, e.g. liquid water, water-based solutions, oils, etc., to pass through / transmit / permeate.

If, for example, the fluid is present as liquid water in the encapsulation and reaches the transmissive layer after an opening of the encapsulation, only the water vapor generated, for example, by the evaporation of this water can penetrate the transmissive layer, which then humidifies the conductive unit (e.g. by condensation of the water vapor on or in the conductive unit to liquid water).

If, for example, the fluid is already present as water vapor in the encapsulation and therefore already reaches the transmissive layer as water vapor after the encapsulation is opened, this water vapor can penetrate the transmissive layer directly. Water vapor condensing on the transmissive layer, on the other hand, must first evaporate again before it can penetrate the transmissive layer. In the case of a fluid already present in the gaseous aggregate state in the encapsulation, e.g. water vapor, it can also be said that the "vaporous parts of the fluid" are the fluid itself.

In a further embodiment of the contact system, the encapsulation can provide (in the undamaged state) separation of the fluid from the transmissive layer. The undamaged state should be understood to provide the encapsulation in an at least basically fluid-tight and vapour-tight condition. In the undamaged state, the encapsulation does not comprise any leaking. Alternatively or additionally, the conductive unit, in particular the conductive layer and/or the electrolyte storage layer, can be provided by the transmissive layer, wherein the transmissive layer can comprise electrically conductive material.

In a further embodiment, the encapsulation can comprise a flexible wrapping, in particular the encapsulation can be made from a water vapour impermeable material, in particular comprising aluminium. The encapsulation can be provided inside of the reservoir in flexible manner.

In a further embodiment, the encapsulation can comprise or is made from a separation layer, in particular from a foil. As for example, the reservoir may be provided by a volume enclosed by walls of the reservoir and the separation layer.

In a further embodiment, the encapsulation can be configured to be broken by snapping the encapsulation. Snapping may cause a rise of pressure inside of the encapsulation which results in breaking of the encapsulation when exceeding a particular resistibility of the encapsulation.

In a further embodiment of the contact system, the contact system can comprise a breaking element configured for breaking the encapsulation upon applying the defined external influence, wherein the breaking element can comprise a perforation portion configured to provide perforation of the encapsulation and can be arranged to perforate the encapsulation upon applying the defined external influence. Optionally, the perforation portion can comprise a tapered end, an edge or a needle.

In a further embodiment, the breaking element can be arranged between the encapsulation and the conductive unit.

In a further embodiment, the breaking element can be arranged between the encapsulation and the fluid absorption unit.

In a further embodiment, the breaking element can be arranged between the encapsulation and the transmissive layer.

In a further embodiment, the breaking element can be arranged in the encapsulation.

The breaking element can be any structural body which - when interacting with the encapsulation - facilitates breaking of the encapsulation.

In a further embodiment, the transmissive layer can comprise a textile or a polymer.

In a further embodiment, the transmissive layer can be provided by a textile layer and/or by a polymeric layer.

In a further embodiment, the transmissive layer can be configured to enable transmission of vapour on the basis of the principle of partial pressure.

In a further embodiment, the transmissive layer can be configured to enable transmission of vaporous parts of the fluid by a defined transmission rate, in particular wherein the transmissive layer comprises perforations (holes) or respectively adjusted fabric and provides the characteristics of a filter.

In one embodiment, the transmissive layer is provided as a layer being permeable for the fluid in a way to provide passing of a defined amount of the fluid in a defined time period. Further, the transmissive layer can provide the functionality of a filter.

In one embodiment, the transmissive layer can be a membrane, in particular a semipermeable membrane.

It is particularly advantageous to provide the transmissive layer and/or the conductive unit, in particular the conductive layer, by a polymeric layer, for example, as a perforated polymer band. The perforated polymeric layer can be glued or hot-pressed directly onto the reservoir, thus saving production steps. The textile, on the other hand, must first be clamped and stretched before being installed in the contact system (e.g. electrode). Thus, the polymeric layer can be better bound to the reservoir (e.g. by gluing or melting, etc.), while the textile layer is merely pressed on.

Another advantage of using a polymeric layer instead of a textile layer to provide the transmissive layer and/or the conductive unit, in particular the conductive layer, is that the conductive textile itself absorbs a lot of fluid, in particular water, and thus transports it to areas where no skin contact takes place. A suitable polymer does not absorb the fluid (e.g. water), so the fluid remains where it has been applied. With a conductive polymer layer, less fluid has to be used. The reservoir can be configured smaller and thus the electrode can be configured smaller and/or flatter. This in turn is more comfortable to wear on the body.

Another advantage of using a polymeric layer instead of a textile layer to provide the transmissive layer and/or the conductive unit, in particular the conductive layer, is that the polymeric layer can be configured as a stiffer conductive porous material compared to a flexible textile, which simplifies handling, especially during production.

In addition, conductive polymer surfaces can be integrated into the contact unit during an injection molding process.

In a further embodiment, the conductive unit, in particular the conductive layer and/or the electrolyte storage layer, can be provided by the transmissive layer, wherein the transmissive layer comprises electrically conductive material.

In a further embodiment, the transmissive layer can be configured to be water vapour permeable and water impermeable with respect to liquid water.

In a further embodiment, the contact system can comprise a contacting layer configured to provide adhesion of the contact system on a surface, in particular on the skin of a person. The contact layer may comprise glue to provide the adhesion.

In a further embodiment, the contacting layer can surround the conductive unit, in particular the conductive layer, or the contacting layer can be provided by the conductive unit, in particular the conductive layer.

In a further embodiment, the reservoir can be provided by a housing, in particular flexible or elastic housing. As for example, the reservoir may comprise basically stiff side walls and a flexible top wall intended to apply the external influence to break the encapsulation.

In a further embodiment, the reservoir can be provided by a flexible cover, in particular a foil. For instance, the reservoir may be provided in form of a pad to be inserted into a respective holder or receiving portion.

In a further embodiment of the contact system, the fluid comprises at least one of the following: a liquid, in particular a high-viscous liquid, a gel, in particular a hydrogel, a solution, in particular a water-based solution or an alcohol-based solution, an emulsion, or oil.

An example of an alcohol-based solution is a solution consisting of ethanol and water, wherein the proportion of ethanol can vary.

The present invention further relates to a method of activating a contact system according to any one of the previous described embodiments, the method comprising the steps of providing the contact system so that the conductive unit is arranged close to or in contact with the skin of a person, and successively, breaking the encapsulation by applying at least the defined external influence to the encapsulation, so that a wetting of the conductive unit by the fluid is provided in such a way that the electrolyte comprised by the conductive unit is dissolved in the fluid and a solution of the electrolyte is provided to the skin of the person from the conductive unit.

The method to activate the contact system provides the advantage that a user of the contact system is enabled to activate the electrode after attaching the electrode, e.g. on the user's skin.

The present invention further relates to a reservoir for a contact system according to any of the preceding embodiments, the reservoir comprises an encapsulation which (at least in an undamaged state) comprises a fluid and which provides separation of the fluid from the conductive unit. Further, the encapsulation is configured to be broken upon defined external influence/exposure, and the encapsulation and the conductive unit are arranged relative to each other so that - in the event of a break of the encapsulation - wetting of the conductive unit by the fluid is provided.

In a further embodiment, the reservoir can be provided by a flexible cover.

The present invention further relates to a wearable element, in particular to a piece of clothing or garment or part thereof, for a contact system according to any of the preceding embodiments, the wearable element comprises a reservoir receiving segment, in particular case, and the conductive unit, wherein the reservoir receiving segment and the conductive unit are arranged relative to another so that - when a reservoir according to any of the preceding embodiments is provided in the reservoir receiving segment and in the event of a break of the encapsulation of the reservoir - wetting of the conductive unit by the fluid is provided.

### BRIEF DESCRIPTION OF THE DRAWINGS

The invention is illustrated in more detail below, purely by way of example, with reference to working examples shown schematically in the drawing. Identical elements are labelled with the same reference numerals in the figures. The described embodiments are generally not shown true to scale and they are also not to be interpreted as limiting the invention.
Figure 1 shows an exemplary embodiment of a conductive unit according to the invention;
Figure 2 shows an exemplary embodiment of a contact system according to the invention;
Figures 3a and 3b show an exemplary embodiment of a contact system according to the invention;
Figures 4a and 4b show an exemplary embodiment of a contact system according to the invention;
Figures 5a and 5b show an exemplary embodiment of a contact system according to the invention;
Figures 6a and 6b show an exemplary embodiment of a contact system according to the invention;
Figure 7 shows an exemplary embodiment of a contact system according to the invention;
Figure 8 shows an exemplary embodiment of a contact system according to the invention; and
Figures 9a and 9b show an exemplary embodiment of a contact system according to the invention provided with a wearable.

### DETAILED DESCRIPTION OF THE DRAWINGS

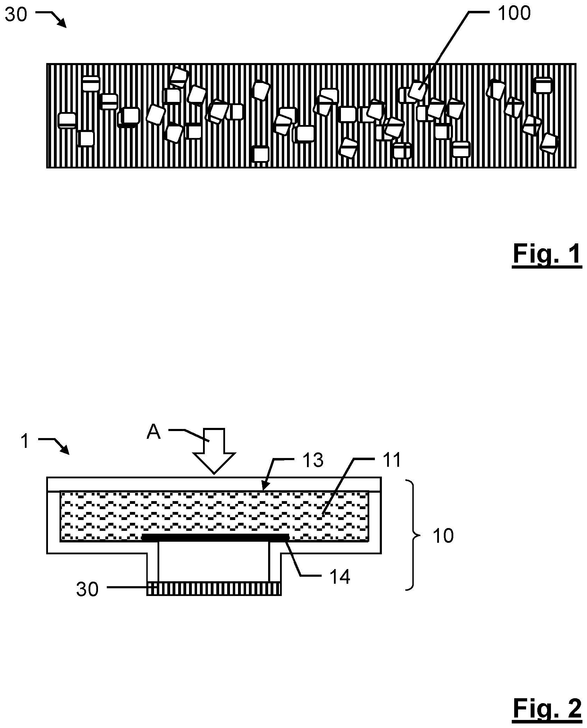

**Figure 1** shows an exemplary embodiment of a conductive unit 30 according to the invention. In the shown embodiment, the conductive unit 30 is configured as a conductive layer, in particular as a conductive textile in whose yarns crystals of an electrolyte 100, e.g. table salt, are present. Thus, the electrolyte 100 has a solid aggregate state until the conductive unit 30 comes into contact with a fluid 11 which dissolves the electrolyte 100.

The conductive unit can, for example, also have a two-layer structure, wherein one layer is a conductive layer, wherein the conductive layer comprises an electrically conductive element, in particular electrically conductive threads or electrically conductive plastic material, in particular made of metal or metallized plastic material, in particular yarns. The conductive layer can comprise the electrolyte in a solid aggregate state or can be provided without an electrolyte. The second layer can be an electrolyte storage layer, wherein the electrolyte storage layer comprises the electrolyte in a solid aggregate state. The electrolyte storage layer can, for example, be configured as a blotting paper in whose fibers salt crystals are provided.

**Figure 2** shows an embodiment of a contact system 1 according to the invention. The contact system 1 comprises a reservoir 10 and the conductive unit 30, wherein the conductive unit 30 only comprises a conductive layer. The reservoir 10 of the contact system 1 here comprises an encapsulation 13. The encapsulation 13 comprises the fluid 11. In the shown embodiment, the encapsulation 13 is provided by the walls of the reservoir 10 as well as by a separation layer 14. The separation layer 14 provides a separation of the fluid 11 from the conductive unit 30 in order to prevent from unwanted wetting of the conductive unit 30. In other words, the separation layer 14 is configured to be tight, in particular gas-tight, with respect to the fluid 11 and acts as a seal.

The separation layer 14 can be a foil or can be manufactured from any material which provides respective sealing properties.

The encapsulation 13, in particular the separation layer 14, is configured to be broken upon defined external influence or exposure. In the shown embodiment, the properties of the encapsulation 13 allow to intentionally damage the encapsulation 13 by applying a particular force to the reservoir 10 or the encapsulation 13. As shown with arrow A, a force can be applied on top of the contact system 1, i.e. on the top wall of the encapsulation 13, which causes a respective force or pressure on the separation layer 14. The separation layer 14 is configured to break upon exposure of the a defined force or stress.

With such configuration, a contact system 1 can be provided which can be activated on demand, i.e. when pressing on the contact system 1 from top. Activation in this context should be understood to start and provide wetting of the conductive unit 30 due to the fluid 11 released from the opened encapsulation 13.

The contact system 1 is arranged on an object 5. The object 5 can be the skin of a person. The contact system 1 is arranged so that the conductive unit 30 is in contact with the object 5. In particular, the contact system 1 adheres on the object 5 by means of fixing means which may be provided by particular glue and/or by a respective contacting layer (not shown).

The (electrically) conductive unit 30 is configured to receive and transmit electrical signals which are provided by the object 5. By that, the contact system 1 provides a system to obtain electrical signals from the object. In particular, in case the object 5 is represented by the skin of a person, the contact system 1 provides transmission of electrical signals generated by particular organs, like the heart of the person, and/or transmitted by particular nerves or tissue. Such electrical signals can be detected on the skin of the person.

Alternatively or additionally the conductive unit 30 can be configured to transmit electrical signals to the object 5. Such configuration can for instance be used to provide stimulation of the object 5, e.g. of a muscle.

An electric conductor can be connected to the conductive unit 30 in order to transmit the collected signals to a respective receiving unit, e.g. to an ECG (electrocardiogram) device, or to the object 5.

As a result of the wetting of the conductive unit 30, the electrolyte 100 present in the conductive unit 30 dissolves in the fluid 11, whereby a solution of the electrolyte 100 is present in the conductive unit 30. Since the conductive unit 30 is in contact with the skin, this electrolyte solution is formed almost directly on the skin 5 of the person, which is why the electrolyte solution has a very high electrolyte concentration directly on the skin 5. The electrolyte concentration on the skin 5 can further be increased if the used fluid 11 already comprises an electrolyte.

In the case of a two-layer structure of the conductive unit 30 with conductive layer and electrolyte storage layer, the conductive unit 30 is arranged on the contact system 1 in such a way that the conductive layer is in contact with the skin and the electrolyte storage layer is present between the encapsulation 13, in particular the separation layer 14, and the conductive layer. Thus, after the fluid exits the encapsulation, the electrolyte storage layer is wetted first, causing the stored electrolyte to dissolve and the resulting electrolyte solution to wet the conductive layer and reach the skin through it.

In order to further increase the electrolyte concentration on the skin, the conductive layer can optionally contain the solid electrolyte, which is dissolved by the electrolyte solution provided by the wetted electrolyte storage layer and thus further increases the concentration of the electrolyte in the electrolyte solution.

Due to this high electrolyte concentration directly on the skin 5, not only is an electrical conductivity between the object 5 and the conductive unit 30 and/or inside of the conductive unit 30 provided, but a reduction of a skin resistance and thus an improved transmission of electrical signals between the skin 5 of the person and the contact system 1 is also provided.

This improved transmission of electrical signals can be seen, for example, in the fact that the signal intensity of an ECG is higher respectively the signals are less noisy respectively fewer interference artifacts occur, which facilitates the evaluation of the ECG, since among other things signals can be assigned more clearly.

This effect cannot be observed at all or only to a much lesser extent with electrodes of the prior art, since in these there is a significantly reduced electrolyte concentration directly on the skin. This is because with prior art electrodes, if an electrolyte source is present at all, the electrolyte solution must diffuse through various layers, membranes, sponges etc., which can lead to a reduction in the electrolyte concentration directly on the skin, as the electrolyte can stick to the layers, membranes, sponges, etc.

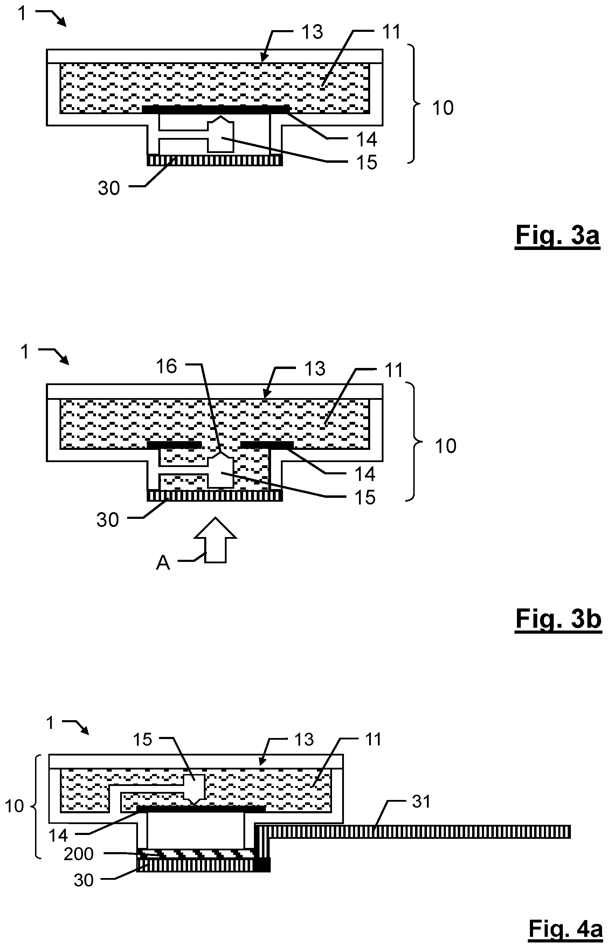

**Figures 3a** and **3b** show a further embodiment of a contact system 1 according to the invention. In contrast to the embodiment of **Figure 2****,** the contact system 1 comprises a breaking element 15.

In particular, the conductive unit 30 is provided by a textile having electrically conductive threads, e.g. the electrically conductive threads are woven in the textile.

The breaking element 15 is arranged between the encapsulation 13, in particular the separation layer 14, and the conductive unit 30. Hence the breaking element 15 is arranged in a bottom part of the electrode 1, which part is intended to be directed towards the object in use.

The breaking element 15 comprises a perforation portion (sharp edge) on its top side directed towards the separation layer 14 for damaging, perforating or breaking the separation layer 14. In an alternative embodiment the perforation portion can be embodied as a needle-like extension for damaging, perforating or breaking the separation layer 14.

The contact system 1 according to the present embodiment is provided as an embodiment intended to be activated before arranging the contact system 1 on the object. Thanks to the breaking element 15, activation can be realised by applying comparatively low pressure or force from the bottom side as depicted by the arrow A. As can be seen in **Figure 3b****,** the separation layer 14 is broken after applying a defined external pressure to the breaking element 15. Hence, activation can be performed easier by applying less pressure.

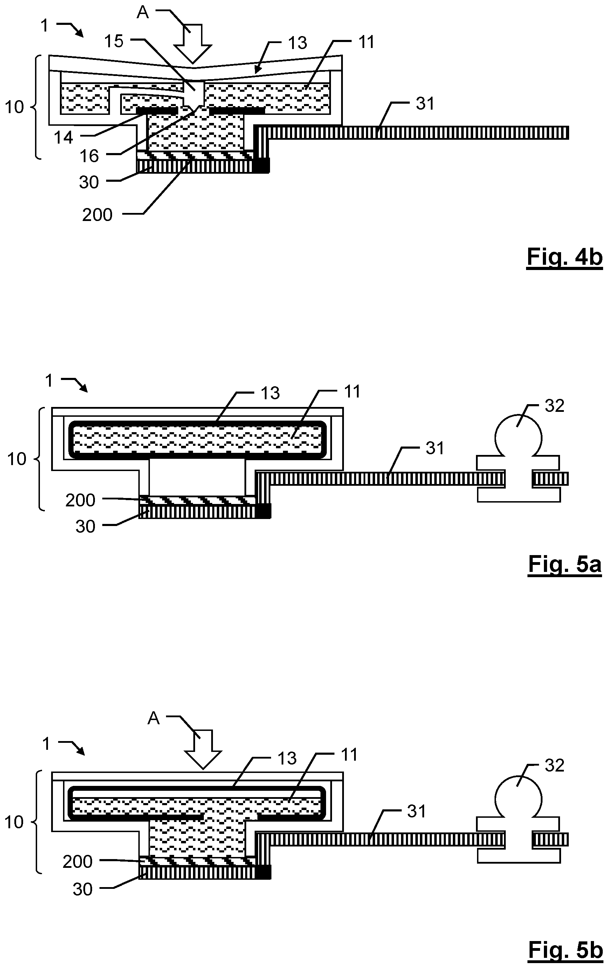

**Figures 4a** and **4b** show a further embodiment of a contact system 1 according to the invention. In contrast to the embodiment of **Figures 3a** and **3b****,** the breaking element 15 is arranged on the opposite side of the separation layer 14. Here, the breaking element 15 is arranged inside of the encapsulation 13.

The breaking element 15 comprises a perforation portion 16 configured to provide perforation of the encapsulation 13, in particular of the separation layer 14, and arranged to perforate the separation layer 14 upon applying the defined external influence on top of the contact system 1.

As can be seen from **Figure 4b****,** the activation force can be applied on the top surface of the encapsulation as shown with arrow A. The top surface is made elastic to provide bending of the surface. By that, when applying the activation force, the top surface of the encapsulation 13 first touches the breaking element 15 and, second, moves the breaking element 15 towards the separation layer 14 to provide interaction of the perforation portion 16 with the separation layer 14. In the course of onward moving of the breaking element 15, the separation layer 14 becomes intentionally damaged, i.e. perforated, broken or cracked. Consequently, the sealing properties of the separation layer 14 are lost and the fluid 11 can then pass the separation layer 14 to get in touch with the fluid absorption unit / layer 200.

In the embodiment shown, the fluid absorption unit 200 is configured as an absorbent sponge, which is arranged between the reservoir 10 and the conductive unit 30. The absorbent sponge 200 ensures that the fluid 11, e.g. liquid water, is stored in a controlled manner so that it does not flow off immediately, but is gradually released to the conductive unit 30 or gradually wets the conductive unit 30. The sponge 200 absorbs the water from the reservoir 10 respectively from the encapsulation 13 as soon as it is released and holds it directly at the interface to the conductive unit 30 (also called textile 30). By that, controlled wetting of the conductive unit 30 is started and/or provided. In this way, a constant electrolyte concentration is present at the skin 5 for the entire duration of the application / measurement.

In other words, the encapsulation 13 is filled with a controlled amount of salt water 11 having a predefined electrolyte concentration. The film 13 encapsulates the water 11 and thus prevents premature leakage. The construction is designed so that the water 11 is only released when targeted pressure is applied to the electrode 1. Pressure on the electrode causes the encapsulation 13 to burst and the contained brine / salt water 11 is released onto the sponge 200 underneath. The sponge 200 immediately absorbs the salt water 11 and transfers it to the textile 30 underneath. This ensures a targeted moistening of the textile 30, causing the solid salt 100 in the textile 30 to dissolve in the salt water 11, whereby an electrolyte solution with a very high electrolyte concentration is present directly on the skin 5. This ensures not only that the electrode 1 remains conductive throughout the measurement, but that transmission of the electrical signals is improved (which can be seen, for example, in more intense signals, less noise, etc.).

Activation (e.g. triggering the breaking mechanism) from above provides the advantage that the contact system 1 (e.g. the electrode) can also be activated if activation was forgotten before the contact system 1 was attached to the skin (e.g. sticking the electrode on). A healthcare provider or a patient can thus activate the contact system 1 (e.g. the electrode) retrospectively.

The contact system 1 also comprises an electrical conductive wire 31 which is connected to the conductive unit 30, in particular the conductive layer of the conductive unit 30. The electrical conductive wire 31 provides connection of the electrode 1 to an external device like an ECG device. The shown embodiment of the electrode 1 is intended to be attached to the skin 5 of a patient during a short application, such as a resting or exercise ECG, and to be easily removable after the measurement has been completed. The materials used and the structure of the electrode 1 are configured in such a way that a targeted, one-time release of liquid 11, e.g. water, takes place, which provides a dissolution of the solid electrolyte 100 in the conductive unit 30 and thus ensures an improved conductivity during the measurement.

The conductive textile 30, which establishes the electrical contact for the ECG measurement, lies directly on the skin. The textile 30 is specially designed for short-term applications and requires rapid, targeted moistening to achieve optimum conductivity.

The targeted bursting of the encapsulation 13 in combination with the continuous release of the salt water 11 from the sponge 200 ensures humidification and therefore the conductivity of the electrode 1 for exactly the desired measurement duration. The design is ideal for short-time applications such as stress ECGs, where quick application and removal of the electrode 1 is required without the need for a permanent supply of moisture.

**Figures 5a** and **5b** show a further embodiment of a contact system 1 according to the invention. In contrast to the embodiment of **Figure 2****,** the encapsulation 13 is here provided by a flexible cover which, in an undamaged state (see **Figure 5a****),** encloses the fluid 11. Hence, the encapsulation 13 is provided in form of a pad having the fluid 11 inside.

Furthermore an electrical conductor 31 is connected to the conductive unit 30, in particular the conductive layer of the conductive unit 30. An interface or pin 32 is connected to the conductor 31 in order to provide easy connection with a measuring or stimulation device.

The pad 13 is configured to be broken by applying a pressure on the top of the electrode 1 (see **Figure 5b****).** The pad 13 preferably gets damaged by the edges provided in the housing of the reservoir 10.

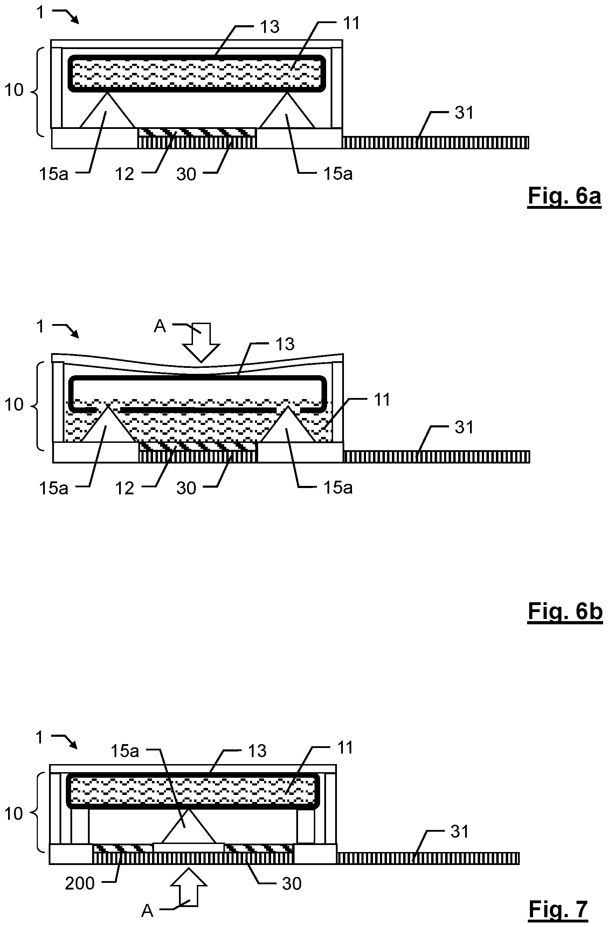

**Figures 6a** and **6b** show a further embodiment of a contact system 1 according to the invention. The contact system 1 comprises a reservoir 10 and a conductive unit 30.

The reservoir 10 comprises a transmissive layer 12 which is configured to provide defined permeation of vapourous parts of the fluid 11 and comprises an encapsulation 13 which encloses the fluid 11. The transmissive layer 12 is provided as a layer being permeable for fluid vapour but being impermeable for liquid fluid, in particular when the fluid is water or a water-based solution. The transmissive layer 12 is designed so that a defined amount of vapour transmits the layer 12 in a defined time period.

The transmissive layer 12 can be provided by a respectively permeable textile or by a perforated polymeric layer.

The encapsulation 13 is provided by a fluid-tight sheath (cover) which provides gas-tight enclosure of the fluid 11. The contact system 1 also comprises at least two breaking elements 15a which here are provided in form of pyramidal elevations with peaked ends. The encapsulation 13 is arranged on top of the breaking elements 15a. The cover of the encapsulation 13 is configured so that the encapsulation 13 is not damaged by the breaking elements 15a and maintains its fluid-tight property in an inactivated state, i.e. in particular as long as there is not applied an external activating force A from top.

**Figure 6b** shows the contact system 1 after activation by applying a pressure on a top wall of the reservoir 10. By applying the pressure in a direction according to arrow A, the encapsulation 13 is pressed against the breaking elements 15a and is cracked by interaction with the breaking elements 15a. As a result, the fluid 11 flows out of the encapsulation 13 and contacts the transmissive layer 12. The fluid 11 provides wetting of the transmissive layer 12 and vapour-like parts of the fluid passes through the transmissive layer 12 and cause wetting of the contact unit 30.

Because the conductive unit 30 is moistened only via the vaporous parts of the fluid 11 diffusing through the transmissive layer 12, the wetting of the conductive unit 30 and thus the dissolution of the solid electrolyte 100 in the conductive unit 30 is significantly slowed down compared to the previously described embodiments without transmissive layer 12. The embodiment shown here is therefore suitable for long-term applications of the contact system 1, since the conductive unit 30 can be moistened over a much longer period of time.

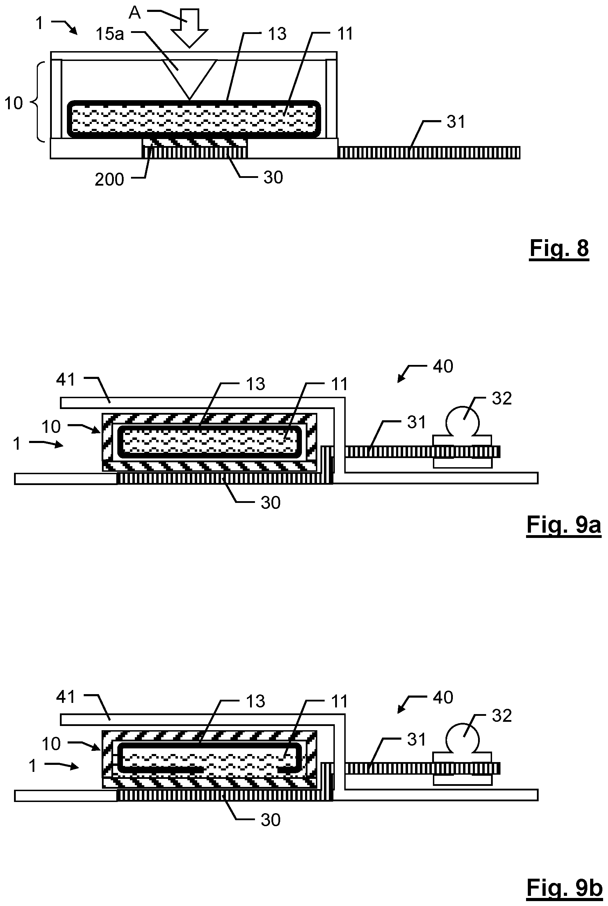

**Figures 7** and **8** show further embodiments of a contact system 1 according to the invention. Both embodiments comprise an activation system which is provided by an encapsulation 13 and a breaking element 13 for perforating the encapsulation 13 upon applying at least a defined activation force (depicted by arrow A). The embodiments differ in the arrangement of the breaking element 15a and in size and configuration of the fluid absorption layer 200 and the conductive unit 30.

According to **Figure 7** the breaking element 15a is arranged between the encapsulation 13 and the conductive unit 30 and the electrode 1 is intended to be activated by pressing from below, i.e. on side of the contacting unit before arranging the electrode 1 on the skin of a person.

According to **Figure 8** the encapsulation 13 is arranged between the breaking element 15a and the fluid absorption layer 200 respectively the conductive unit 30. Here, the electrode 1 is intended to be activated by pressing from top, i.e. directly on the top wall of the reservoir 10 on which the breaking element 15a is mounted. The electrode 1 of **Figure 8** can easily be activated after being arranged on the skin of a person.

**Figures 9a** and **9b** show a further embodiment of a contact system 1 according to the invention. The contact system 1 comprises a reservoir 10 which is provided by a flexible cover, in particular by a foil or textile. Here, the reservoir 10 is preferably embodied as a pad.

The pad 10 comprises an encapsulation 13. A fluid 11 is provided inside of the encapsulation 13. The encapsulation 13 is configured to be tight with respect to the contained fluid 11. Moreover, the encapsulation 13 is configured to be breakable upon exposure of an external force and, by that, to provide outflowing of the fluid (see **Figure 9b****),** i.e. the encapsulation 13 can be broken when pressing on the encapsulation 13 and/or when snapping, squeezing or cracking the encapsulation 13. The conductive unit 30 is moistened when the encapsulation 13 is broken because the pad 10 is permeable to the fluid 11. The pad 10 can optionally also be designed like the fluid absorption unit 200 described above.

The contact system 1 also comprises a conductive unit 30 which is connected with a wire 31 to provide electrical conductivity to and from a connecting pin 32.

The conductive unit 30 is provided by a wearable element 40, i.e. by a piece of clothing. In particular, the conductive unit 30 is provided by electrical conductive threads which are woven in the wearable element 40. Alternatively, the conductive unit 30 can be provided by an additional textile or polymeric layer which is glued or sewn on the wearable element 40.

The wearable element 40 comprises a reservoir receiving segment 41, in particular case or bag. The bag 41 is configured to receive the reservoir 10. In particular, the reservoir 10 can be inserted into the reservoir receiving segment 41. The encapsulation 13 is preferably broken after inserting in order to provide wetting of the conductive unit 30 on demand.

The reservoir receiving segment 41 and the conductive unit 30 are arranged relative to another so that - when a reservoir 10 is provided in the reservoir receiving segment 41 and in the event of a break of the encapsulation 13 of the reservoir 10 - wetting of the conductive unit 30 by the fluid 11 is provided.

The reservoir 10 can be removed from the reservoir receiving segment 41 when the fluid 11 is consumed and wetting of the conductive unit 30 can no longer be provided. Moreover, a further (new, undamaged) reservoir 10 (pad) can then be inserted into the bag 41 to continue wetting of the conductive unit 30.

The wearable element 40 can be provided as a shirt or pants or may be any other wearable component intended to be worn and having contact with the body of a person.

Although the invention is illustrated above, partly with reference to some preferred embodiments, it must be understood that numerous modifications and combinations of different features of the embodiments can be made. All of these modifications lie within the scope of the appended claims.

## Claims

1. Conductive unit (30) for a contact system (1), in particular electrode, wherein the conductive unit (30) is provided to be contacted with a skin of a person such that an electrical contact to the skin of the person by the contact system (1) is provideable,
**characterised in that**
the conductive unit (30) comprises an electrolyte (100) having a solid aggregate state.

2. Conductive unit (30) according to claim 1, wherein the conductive unit (30) comprises a conductive layer, wherein the conductive layer
• comprises an electrically conductive element, in particular electrically conductive threads or electrically conductive synthetic material, in particular made of metal or metallized synthetic material, in particular yarns, and/or
• is provided to be contacted with the skin of the person,
in particular wherein the conductive layer comprises the electrolyte (100) having a solid aggregate state.

3. Conductive unit (30) according to claim 2, wherein the conductive unit (30) further comprises an electrolyte storage layer, wherein the electrolyte storage layer comprises the electrolyte (100) having a solid aggregate state.

4. Conductive unit (30) according to one of the preceding claims, wherein the electrolyte (100) is dissolvable in a fluid (11) by wetting or humidifying the conductive unit (30) with the fluid (11) to provide a solution of the electrolyte (100) so that the solution of the electrolyte (100) is providable to the skin of the person (5) from the conductive unit (30), in particular wherein the electrolyte (100) is already comprised by the fluid (11) prior to the wetting or humidifying of the conductive unit (30) with the fluid (11).

5. Conductive unit (30) according to one of the preceding claims, wherein the electrolyte (100) comprises at least one alkaline metal salt, in particular sodium chloride or potassium chloride, and/or at least one alkaline earth metal salt, in particular magnesium chloride or calcium chloride.

6. Conductive unit (30) according to claim 2 or 3, wherein a hydrophobicity of the conductive layer is reduced by the electrolyte (100) compared to a hydrophobicity of a conductive layer comprising the same material as the conductive layer but without the electrolyte (100), in particular wherein the hydrophobicity of the conductive layer is a measure of a size of a contact angle between the conductive layer and the fluid (11) which can be determined by means of static contact angle measurement.

7. Method for producing a conductive unit (30) according to any one of the claims 1 to 6, the method comprising the step of applying the electrolyte (100) to a conductive unit precursor, wherein the electrolyte (100) is applied to the conductive unit precursor
• by mechanical application of the electrolyte (100) being in the solid aggregate state, and/or
• by means of a production fluid, in particular wherein the electrolyte (100) is dissolved in the production fluid,
in particular wherein the conductive unit precursor corresponds to the conductive layer without the electrolyte (100) and/or the electrolyte storage layer without the electrolyte (100).

8. Method according to claim 7, wherein the production fluid comprises at least one of the following:
• a gas, in particular nitrogen or air,
• a liquid, in particular water,
• a gel, in particular a hydrogel,
• a solution, in particular a water-based solution or an alcohol-based solution,
• an emulsion, or
• oil.

9. Method according to claim 7 or 8, wherein, if the electrolyte (100) is applied to the conductive unit precursor dissolved in the production fluid, the method further comprising the step of evaporating the production fluid, in particular wherein the evaporation of the production fluid is provided by
• exposing the conductive unit precursor to ambient air,
• exposing the conductive unit precursor to a gas flow,
• applying a negative pressure to the conductive unit precursor, and/or
• increasing a temperature around the conductive unit precursor.

10. Contact system (1), in particular electrode, for providing an electric contact to the skin of a person (5), the contact system (1) comprising
• a conductive unit (30) according to any one of the claims 1 to 6, and
• a reservoir (10), the reservoir (10) comprises an encapsulation (13), wherein the encapsulation (13)
∘ comprises a fluid (11),
∘ provides separation of the fluid (11) from the conductive unit (30), and
∘ is configured to be broken upon defined external influence,
wherein the conductive unit (30) is arranged relative to the encapsulation (13) so that - in the event of a break of the encapsulation (13) - wetting of the conductive unit (30) by the fluid (11) is provided.

11. Contact system (1) according to claim 10, wherein the contact system (1) further comprises a fluid absorption unit (200), in particular a sponge, wherein the fluid absorption unit (200) is arranged between the conductive unit (30) and the encapsulation (13), so that - in the event of a break of the encapsulation (13) - wetting of the fluid absorption unit (200) by the fluid (11) is provided, so that the fluid absorption unit (200) absorbs the fluid (11) and then successively releases the fluid (11) to the conductive unit (30).

12. Contact system (1) according to claim 10, wherein the reservoir (10) comprises a transmissive layer (12), wherein
• the transmissive layer (12) is configured to provide defined transmission of vaporous parts of the fluid (11) with a defined transmission rate, and
• the encapsulation (13), the transmissive layer (12) and the conductive unit (30) are arranged relative to each other so that - in the event of a break of the encapsulation (13) - wetting of the transmissive layer (12) by the fluid is provided and humidifying of the conductive unit (30) by transmission of the vaporous parts of the fluid (11) through the transmissive layer (12) is provided.

13. Contact system (1) according to anyone of the claims 10 to 12, wherein the contact system (1) comprises a breaking element (15) configured for breaking the encapsulation (13) upon applying the defined external influence, wherein the breaking element (15) comprises a perforation portion (16) configured to provide perforation of the encapsulation (13) and arranged to perforate the encapsulation (13) upon applying the defined external influence, in particular wherein the perforation portion (16) comprises a tapered end, an edge or a needle.

14. Contact system (1) according to anyone of the claims 10 to 13, wherein the fluid (11) comprises at least one of the following:
• a liquid, in particular a high-viscous liquid,
• a gel, in particular a hydrogel,
• a solution, in particular a water-based solution or an alcohol-based solution,
• an emulsion, or
• oil.

15. Method of activating a contact system (1) according to any one of the claims 10 to 14, the method comprising the steps of
• providing the contact system (1) so that the conductive unit (30) is arranged close to or in contact with the skin of a person (5), and
• successively, breaking the encapsulation (13) by applying at least the defined external influence to the encapsulation (13), so that a wetting of the conductive unit (30) by the fluid (11) is provided in such a way that the electrolyte (100) comprised by the conductive unit (30) is dissolved in the fluid (11) and a solution of the electrolyte (100) is provided to the skin of the person (5) from the conductive unit (30).
